# EUROPEAN PATENT SPECIFICATION

(11) **EP 3 852 696 B1**
(45) Date of publication and mention of the grant of the patent: **26.07.2023**
(21) Application number: 19863052.7
(22) Date of filing: 17.09.2019
(51) Int. Cl.: A61F 5/56

(54) **ANTI-SNORING DEVICE**
ANTISCHNARCHVORRICHTUNG
DISPOSITIF ANTI-RONFLEMENT

(30) Priority: 18.09.2018 SE 1851107
(43) Date of publication of application: 28.07.2021
(73) Proprietor: Sleeptight AB, 224 75 Lund (SE)
(72) Inventor: WALLIN, Marianne, 224 75 Lund (SE)
(74) Representative: Ström & Gulliksson AB
(86) International application number: PCT/SE2019/050877
(87) International publication number: WO 2020/060472

(56) References cited:
- WO-A1-2009/104996
- DE-U1- 20 105 290
- DE-U1- 20 105 290
- DE-U1- 20 121 093
- DE-U1- 20 121 093
- US-A1- 2008 223 377
- US-A1- 2008 223 377
- US-A1- 2016 051 391
- US-B1- 6 668 834

## Description

### Technical Field

The present invention relates to devices for maintaining open airways during sleep, to devices that prevents or at least reduces snoring and more specifically to anti-snoring devices that are attached to the neck of a person and that are to be worn during sleep.

### Background

Snoring is a problem for a large number of people and may cause associated problems with sleep. Snoring is also related to obstructive sleep apnoea, which is a more serious condition where breathing ceases for short intermittent periods of time. Snoring is generally caused by a narrowing of the airways, which causes vibrations and consequently noise and/or restricted breathing during sleep. Such a narrowing of the airways may be attributed to several factors, for instance may allergies, nasal congestions, throat weakness and obesity all contribute to the occurrence of snoring. Head and jaw position will also affect the airways and it is thus important that these are kept in a beneficial position during sleep to help avoid snoring.

No single treatment exists today that will completely remove snoring; however there are a number of ways that snoring can be reduced. It is common to use orthopedic pillows that helps to keep the head in a position where snoring is reduced, and there are also dental appliances that may be used to keep the lower jaw in a slightly advanced position in relation to the upper jaw which also reduces the risk of snoring. There are furthermore contraptions that can be strapped around the neck while sleeping and that maintains the jaw and/or the head in a position where snoring is reduced. Additionally, there are also more complex and uncommon alternatives such as using continuous positive airway pressure machines and even surgery. The latter being more uncommon and a drastic solution for only the most severe cases. A common problem with the available solutions today are that, while they may reduce snoring, the contraptions themselves may cause trouble sleeping as they require the person using it to keep a certain position or because they restrict motion during sleep.

It is therefore desired to provide a device that will reduce or stop snoring and that is comfortable to wear during sleep, and that allows a greater span of different sleeping positions.

Related background art can be found in WO 2009 104996 A1. The document DE 201 05 290 U1 discloses the preamble of claim 1.

### Summary

It is thus an object of the teachings herein to provide an improved anti-snoring device overcoming or mitigate at least some of the disadvantages of prior art solutions.

According to a first aspect, an anti-snoring device is provided for attachment to the neck of a user. The anti-snoring device comprises a harness made out of a flexible material, the harness comprising a retaining portion. The anti-snoring device further comprises an insert configured to be arranged in the retaining portion of the harness, the insert comprising a base portion configured to face the neck of the user, and first and second surfaces defining a laterally tapering shape of the insert with increasing distance from the base portion. The anti-snoring device will thus provide improved comfort to the user as the tapering shape will reduce the likelihood of the device being pushed out of position during sleep. This will improve the usability and functionality of the device, as a user will tend to feel less limited in terms of attainable sleeping positions while still achieving satisfactory reduction in snoring.

The base portion of the insert may further comprise a central recess. The recess in the base portion helps maintain the insert in the correct position as it improves the conformity to the shape of the user's neck or more specifically to the shape of the protrusion of the larynx.

In one embodiment, a third surface of the insert is provided between each of the first and second surfaces and the base portion has a curved shape. The curved shape of the third surface improves the comfort for the user, as it is this surface that will form the contact surface with the chin of the user as well as with the upper part of the sternum.

The third surface may define the laterally projected profile of the insert providing an essentially elliptical or oval profile with the exception of one essentially flat section defined by the base portion.

The base portion may further comprise an essentially central hole, improving the stability of the insert when mounted against the larynx of the user.

In one embodiment, the insert is hollow, reducing the required material for producing the insert as well reducing the weight thereof.

In yet another embodiment, the first and second surfaces are essentially elliptical or oval.

In one embodiment, the vertical height of the insert is in the range of 65 to 80 mm, preferably in the range of 70 to 75 mm, or even more preferred approximately 72 mm. The above-specified height ranges will suit a large span of users and make sure that the lower jaw and the head of the user in the correct position.

In an embodiment, the horizontal length of the insert as measured from the base portion is in the range of 55 to 70 mm, preferably in the range of 60 to 65 mm, or even more preferred approximately 62 mm. The horizontal length will ensure that sufficient support is provided while avoiding that the anti-snoring device will not protrude to far outside the chin of the user.

In one embodiment, the horizontal width of the insert is in the range of 55 to 75 mm, preferably in the range of 60 to 70 mm, or even more preferred approximately 75 mm, preventing or at least reducing protrusion laterally outside the lower jaw of the user.

In one embodiment, the diameter of the central hole in the base portion is in the range of 25 to 40 mm, preferably in the range of 30 to 35 mm, or even more preferred approximately 32 mm.

The base portion may have the shape of an outwardly convex curve swept in an essentially elliptical or oval pattern, the curve in one end transitioning to a third surface of the insert and in the other end to the recess. Thereby will the base portion form a contact surface essentially corresponding to that of a torus or toroid. Such a contact surface helps keep the insert in the correct position as well as improves the overall comfort when wearing the device.

The harness may comprise two elongated straps attached to either lateral side of the retaining portion, the straps are configured to hold the anti-snoring device in place around the neck of the user.

The harness may comprise two insert holding straps, the straps being configured to hold the insert in place in the retaining portion.

Further advantages and embodiments are disclosed below and in the appended claims.

### Brief Description of the Drawings

Embodiments of the teachings herein will be described in further detail in the following with reference to the accompanying drawings which illustrate non-limiting examples on how the embodiments can be reduced into practice and in which:
Fig. 1 shows a lateral view of an anti-snoring device according an embodiment,
Fig. 2 shows a lateral view of an insert according to an embodiment,
Fig. 3 shows a rear view of an insert according to an embodiment,
Fig. 4 shows a vertical view of an insert according to an embodiment, and
Fig. 5 shows a perspective view of an insert according to an embodiment.

### Detailed description

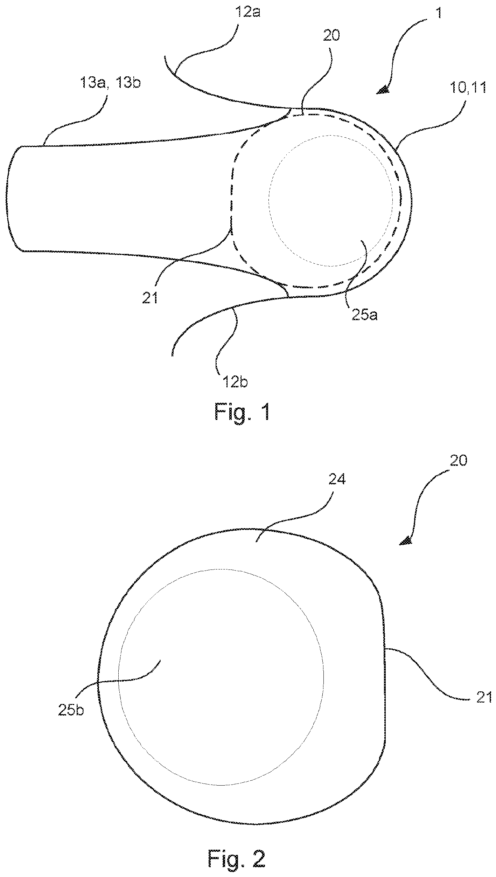

The disclosed embodiments will now be described more fully hereinafter with reference to the accompanying drawings, in which certain embodiments of the invention are shown. Like numbers refer to like elements throughout.
Firstly will reference be made to Fig 1 in which an anti-snoring device 1 is shown. The device 1 is shown from the side or laterally, i.e. as seen from the side a user wearing the device 1. The device comprises a harness 10 into which an insert 20 is placed. When observing the device 1 from the side as in Fig. 1, the insert 20 would normally be hidden from view by the harness 10 as is indicated by the dashed line of the insert 20. It is however made visible for illustrating its placement in relation to the harness 10. The purpose of the harness is to hold the insert 20 in place as well as to provide a cushioning and stabilizing effect. The harness is preferably made out of a flexible, washable material such as a textile material, e.g elastane, spandex or similar.

The harness 10 comprises a retaining portion 11 that is configured to hold the insert 20. The retaining portion 11 is essentially a pocket formed in the portion of the harness 10, that is to be arranged on the forward facing side of the users neck, preferably over the larynx. The shape of the retaining portion 11 is thus adapted to match the shape of the insert 20, to ensure that the insert 20 is unable to rotate or shift while placed in the retaining portion 11.

The harness 10 further comprises two elongated straps 13a, 13b, which are intended to be placed around the neck of the user and attached to each other for instance by means of strips of Velcro^{®}. Other means of attaching the straps to each other are of course also possible, such as buttons etc. The straps 13a, 13b connects to each lateral side of the retaining portion 11.

In order to secure the insert 20 in the retaining portion 11, so that it does not fall out when the device 1 is removed from the neck, holding straps 12a, 12b may be provided on the harness 10. These preferably connects to the upper and lower portion of the retaining portion 11 respectively, and folds over the insert 20, so that they can be attached to each other by means of for instance Velcro^{®}. While the harness 10 is configured to hold the insert 20 in place, it is the insert 20 that is the actual structural part of the anti-snoring device 1.

The user may put on the device 1 around the neck, preferably with the retaining portion 11 and the insert 20 arranged over the larynx of the user. Thereby, the anti-snoring device 1 will hold the head and the mandible of the user in a position that facilitates keeping the airways open/unrestricted. This will reduce the likelihood of the occurrence of snoring. In this process, it is the insert 20 that forms the structural support below the chin of the user, which restricts the mandible and the head of the user from attaining a position that is unfavorable in terms of snoring. In other words, the insert 20 will prevent a position of the lower jaw that is too close to the sternum or the chest, as such positions are known to induce snoring.

The insert 20 separated from the harness 10 is shown from a lateral perspective in Fig. 2. I.e. as it would appear when observing a user wearing the device 1 from a lateral perspective. The insert 20 is made from a flexible but relatively firm material such as a polymeric material and is preferably hollow. It may also be made from a lightweight polymeric foam material, or from another material as well. The overall weight of the insert 20 is preferably less than 20 grams. The vertical height of the insert 20 may be in the range of 65 to 80 mm, preferably in the range of 70 to 75 mm, or even more preferred approximately 72 mm. The horizontal length of the insert 20 as measured from the base portion 21 to the opposite end may be in the range of 55 to 70 mm, preferably in the range of 60 to 65 mm, or even more preferred approximately 62 mm.

The insert 20 is preferably made in one piece and does not have any joints or seams that could compromise the comfort of the user. The insert 20 comprises a base portion 21, the base portion 21 is seen from the side in Fig. 2 as an essentially flat section on the otherwise essentially elliptical or oval profile. The base portion 21 is configured to form the part of the insert 20 that will rest against the larynx of the user. For providing comfort and to facilitate positioning may the base portion 21 comprise a central recess 22 and in the recess may a hole 26 be arranged. The base portion 21 with the recess 22 and the central hole 26 provides a structural contact surface that essentially resembles the contact surface that a torus or toroid-shaped structure would have. I.e. the base portion 21 has the shape of an outwardly convex curve that is swept in an essentially elliptical or oval pattern. The curve, or more specifically the swept surface defined by the curve, in one end transitions to a third surface 24 and on the other end forms or transitions to the recess 22 or the hole 26. The hole 26 is formed below the most outward part of the base portion 21, i.e. in the recess 22 and thus forms one end of the curve mentioned above.

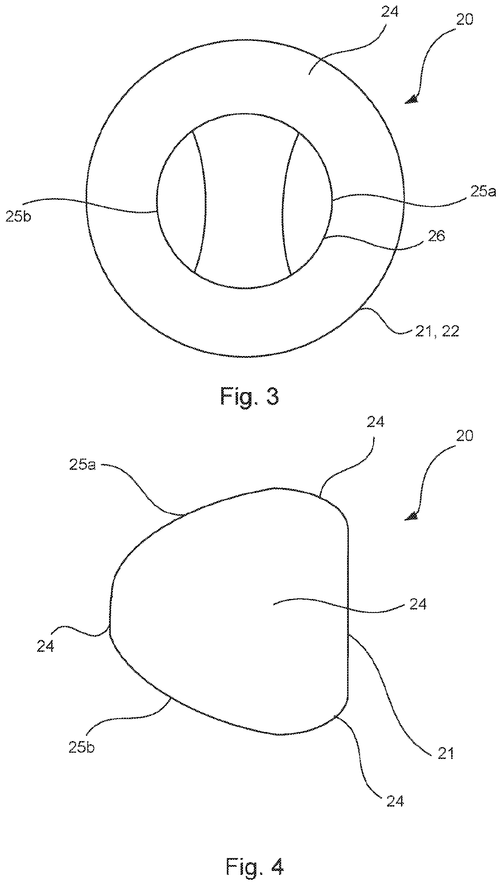

In order to achieve an anti-snoring device 1 that will allow a greater span of sleeping positions than prior art devices, the insert 20 comprises a laterally tapering shape with increasing distance from the base portion 21. I.e., when observed from above or below (as seen in Fig. 4) will the insert 20 appear almost triangular in shape, albeit with significantly curved edges. The laterally tapering shape will restrict interference with the positioning of the insert 20 when the user is for instance lying on the side. Generally, the concept of the tapering shape of the insert 20 is to minimize its protrusion laterally outside of the lower jaw of the user, thus reducing the likelihood of it being pushed out of place when the user lies on the side.

The tapering shape is achieved by the provision of first and second surfaces 25a, 25b that are arranged on the laterally facing sides of the insert 20. Each of the first and second surfaces 25a, 25b and the base portion 21 are connected by a third surface 24 that has a curved shape. The third surface 24 thus constitutes the entire remaining surface of the insert 20 between each of the first and second surfaces 25a, 25b and the base portion 21. The third surface 24 defines the profile of the insert 20 as observed laterally and from the front or rear (i.e. as seen in Figs 2 and 3 respectively), providing an essentially elliptical or oval profile from these perspectives. The curved third surface 24 provides comfort to the user as it is this surface that forms the underlying structure of the contact surface for the underside of the chin and the chest/sternum during use.

The first and second surfaces 25a, 25b are inclined in relation to one another and in relation to the base portion 21. This achieves the above-mentioned essentially triangular shape, with the end of the insert 20 that is opposite the base portion 21 being more narrow in the lateral direction than the former.

The first and second surfaces 25a, 25b are both preferably flat surfaces but they may also have a slightly curved surface or have perforations, indentations etc. The first and second surfaces 25a, 25b preferably have an essentially elliptical or oval shape.

Fig. 3 shows a rear view of the insert 20. The elliptical or oval profile is clearly shown, as is the hole 26 that is provided in the base portion 21 of the insert 20. The first and second surfaces 25a, 25b, which are seen through the hole 26, are facing forwards and laterally as the insert 20 is observed from a viewpoint corresponding to that of the larynx during use. The horizontal width, as measured between each lateral most side of the insert 20 may be in the range of 55 to 75 mm, preferably in the range of 60 to 70 mm, or even more preferred approximately 75 mm. The specified widths of the insert 20 keeps the lateral protrusion outside of the mandible of the user to a minimum, or completely avoids protrusion outside the mandible.

The diameter of the central hole 26 in the base portion 21 may be in the range of 25 to 40 mm, preferably in the range of 30 to 35 mm, or even more preferred approximately 32 mm.

Fig. 4 shows the insert 20 as observed vertically, i.e. from above or below. The curved third surface 24 is shown and how it is delimited by the first and second surfaces 25a, 25b as well as by the base portion 21. What is further shown in Fig. 4 is that the third surface 24 continues between each of the thereto-adjoining surfaces 25a, 25b, 21. It is to be noted that the curvature of the third surface 24 may not be constant over the entire insert 20, as portions of the third surface 24 may be more or less curved.

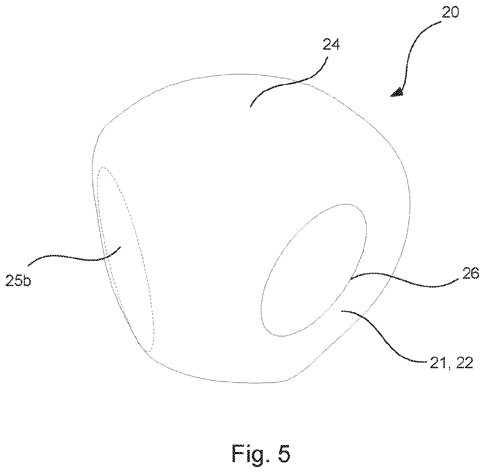

Turning to Fig. 5, where a perspective view of the insert 20 is shown. The base portion 21 is shown along with the thereto provided recess 22 and hole 26. The hollow shape of the insert 21 is also illustrated, which naturally saves material and reduces the weight of the insert 20.

From the description above follows that, although various embodiments of the invention have been described and shown, the invention is not restricted thereto, but may also be embodied in other ways within the scope of the subject-matter defined in the following claims.

## Claims

1. Anti-snoring device (1) for attachment to the neck of a user, said anti-snoring device comprises:
- a harness (10) made out of a flexible material, said harness (10) comprising a retaining portion (11),
- an insert (20) configured to be arranged in the retaining portion (11) of the harness (10) on the forward facing side of the user's neck,
wherein said insert (20) comprises a base portion (21) configured to face the neck of the user, and first and second surfaces (25a, 25b) defining a laterally tapering shape of the insert (20) with increasing distance from the base portion (21), **characterised in that** the base portion (21) of the insert (20) comprises a central recess (22) with a hole (26) providing a torus or toroid-shaped structural contact surface.

2. Anti-snoring device (1) according to claim 1, wherein a third surface (24) of the insert (20) between each of the first and second surfaces (25a, 25b) and the base portion (21) has a curved shape.

3. Anti-snoring device (1) according to claim 2, wherein the curved third surface (24) defines the profile of the insert (20) providing an elliptical or oval profile with the exception of one essentially flat section defined by the base portion (21).

4. Anti-snoring device (1) according to any one of the preceding claims, wherein the insert (20) is hollow.

5. Anti-snoring device (1) according to any one of the preceding claims, wherein the first and second surfaces (25a, 25b) are essentially elliptical or oval.

6. Anti-snoring device (1) according to any one of the preceding claims, wherein the vertical height of the insert (20) is in the range of 65 to 80 mm, preferably in the range of 70 to 75 mm, or even more preferred approximately 72 mm.

7. Anti-snoring device (1) according to any one of the preceding claims, wherein the horizontal length of the insert (20) as measured from the base portion (21) to the opposite end is in the range of 55 to 70 mm, preferably in the range of 60 to 65 mm, or even more preferred approximately 62 mm.

8. Anti-snoring device (1) according to any one of the preceding claims, wherein the horizontal width of the insert (20) is in the range of 55 to 75 mm, preferably in the range of 60 to 70 mm, or even more preferred approximately 75 mm.

9. Anti-snoring device (1) according to claim 1, wherein the diameter of the central hole (26) in the base portion (21) is in the range of 25 to 40 mm, preferably in the range of 30 to 35 mm, or even more preferred approximately 32 mm.

10. Anti-snoring device (1) according to claim 1, wherein the base portion (21) comprises the shape of an outwardly convex curve swept in an elliptical or oval pattern, the curve in one end transitioning to a third surface (24) of the insert (20) and in the other end to the recess (22) with the hole (26) providing the torus or toroid-shaped structural contact surface.

11. Anti-snoring device (1) according to any one of the preceding claims, wherein the harness (10) comprises two elongated straps (13a, 13b) attached to either lateral side of the retaining portion (11), wherein said straps are configured to hold the anti-snoring device (1) in place around the neck of the user.

12. Anti-snoring device (1) according to any one of the preceding claims, wherein the harness (10) comprises two insert holding straps (12a, 12b), said straps (12a, 12b) being configured to hold the insert (20) in place in the retaining portion (11).

## Patentansprüche

1. Antischnarchvorrichtung (1) zur Befestigung am Hals eines Benutzers, wobei die Antischnarchvorrichtung umfasst:
- einen Harnisch (10), gemacht aus einem flexiblen Material, wobei der Harnisch (10) einen Halteabschnitt (11) umfasst,
- einen Einsatz (20), der ausgebildet ist, in dem Halteabschnitt (11) des Harnisch (10) auf der nach vorne gerichteten Seite des Halses des Benutzers angeordnet zu werden, wobei der Einsatz (20) einen Basisabschnitt (21) umfasst, der ausgebildet ist, dem Hals des Benutzers zugewandt zu sein, sowie erste und zweite Oberflächen (25a, 25b), die eine sich mit zunehmendem Abstand von dem Basisabschnitt (21) seitlich verjüngende Form des Einsatzes (20) definieren, **dadurch gekennzeichnet, dass** der Basisabschnitt (21) des Einsatzes (20) eine zentrale Aussparung (22) mit einem Loch (26) umfasst, das eine torus- oder toroidförmige strukturelle Kontaktfläche bereitstellt.

2. Antischnarchvorrichtung (1) nach Anspruch 1, wobei eine dritte Oberfläche (24) des Einsatzes (20) zwischen jeder der ersten und zweiten Oberflächen (25a, 25b) und dem Basisabschnitt (21) eine gekrümmte Form aufweist.

3. Antischnarchvorrichtung (1) nach Anspruch 2, wobei die gekrümmte dritte Oberfläche (24) das Profil des Einsatzes (20) definiert, das ein elliptisches oder ovales Profil mit Ausnahme eines im Wesentlichen flachen Abschnitts bereitstellt, der durch den Basisabschnitt (21) definiert ist.

4. Antischnarchvorrichtung (1) nach einem der vorhergehenden Ansprüche, wobei der Einsatz (20) hohl ist.

5. Antischnarchvorrichtung (1) nach einem der vorhergehenden Ansprüche, wobei die ersten und zweiten Oberflächen (25a, 25b) im Wesentlichen elliptisch oder oval sind.

6. Antischnarchvorrichtung (1) nach einem der vorhergehenden Ansprüche, wobei die vertikale Höhe des Einsatzes (20) im Bereich von 65 bis 80 mm ist, vorzugsweise im Bereich von 70 bis 75 mm ist, oder noch bevorzugter etwa 72 mm ist.

7. Antischnarchvorrichtung (1) nach einem der vorhergehenden Ansprüche, wobei die vom Basisabschnitt (21) bis zum gegenüberliegenden Ende gemessene horizontale Länge des Einsatzes (20) im Bereich von 55 bis 70 mm ist, vorzugsweise im Bereich von 60 bis 65 mm ist, oder noch bevorzugter etwa 62 mm ist.

8. Antischnarchvorrichtung (1) nach einem der vorhergehenden Ansprüche, wobei die horizontale Breite des Einsatzes (20) im Bereich von 55 bis 75 mm ist, vorzugsweise im Bereich von 60 bis 70 mm ist, oder noch bevorzugter etwa 75 mm ist.

9. Antischnarchvorrichtung (1) nach Anspruch 1, wobei der Durchmesser des zentralen Lochs (26) in dem Basisabschnitt (21) im Bereich von 25 bis 40 mm ist, vorzugsweise im Bereich von 30 bis 35 mm ist, oder noch bevorzugter etwa 32 mm ist.

10. Antischnarchvorrichtung (1) nach Anspruch 1, wobei der Basisabschnitt (21) die Form einer nach außen führenden konvexen Kurve aufweist, die in einem elliptischen oder ovalen Muster verläuft, wobei die Kurve an einem Ende in eine dritte Oberfläche (24) des Einsatzes (20) und an dem anderen Ende in die Aussparung (22) mit dem Loch (26) übergeht, das die torus- oder toroidförmige strukturelle Kontaktfläche bereitstellt.

11. Antischnarchvorrichtung (1) nach einem der vorhergehenden Ansprüche, wobei der Harnisch (10) zwei längliche Riemen (13a, 13b) umfasst, die an beiden Seiten des Halteabschnitts (11) befestigt sind, wobei die Riemen ausgebildet sind, die Antischnarchvorrichtung (1) um den Hals des Benutzers in Position zu halten.

12. Antischnarchvorrichtung (1) nach einem der vorhergehenden Ansprüche, wobei der Harnisch (10) zwei Einsatzhalteriemen (12a, 12b) aufweist, wobei die Riemen (12a, 12b) ausgebildet sind, den Einsatz (20) in dem Halteabschnitt (11) in Position zu halten.

## Revendications

1. Dispositif antironflement (1) destiné à être fixé au cou d'un utilisateur, ledit dispositif antironflement comprend :
- un harnais (10) composé d'un matériau flexible, ledit harnais (10) comprenant une partie de retenue (11),
- un insert (20) configuré pour être agencé dans la partie de retenue (11) du harnais (10) sur le côté tourné vers l'avant du cou de l'utilisateur,
dans lequel ledit insert (20) comprend une partie de base (21) configurée pour être tournée vers le cou de l'utilisateur, et des première et deuxième surfaces (25a, 25b) définissant une forme s'amincissant latéralement de l'insert (20) avec une distance croissante à partir de la partie de base (21), **caractérisé en ce que** la partie de base (21) de l'insert (20) comprend un évidement central (22) avec un trou (26) fournissant une surface de contact structurale en forme de tore ou toroïdale.

2. Dispositif antironflement (1) selon la revendication 1, dans lequel une troisième surface (24) de l'insert (20) entre chacune des première et deuxième surfaces (25a, 25b) et la partie de base (21) présente une forme courbée.

3. Dispositif antironflement (1) selon la revendication 2, dans lequel la troisième surface courbée (24) définit le profil de l'insert (20) fournissant un profil elliptique ou ovale à l'exception d'une section essentiellement plate définie par la partie de base (21).

4. Dispositif antironflement (1) selon l'une quelconque des revendications précédentes, dans lequel l'insert (20) est creux.

5. Dispositif antironflement (1) selon l'une quelconque des revendications précédentes, dans lequel les première et deuxième surfaces (25a, 25b) sont essentiellement elliptiques ou ovales.

6. Dispositif antironflement (1) selon l'une quelconque des revendications précédentes, dans lequel la hauteur verticale de l'insert (20) est dans la plage de 65 à 80 mm, de préférence dans la plage de 70 à 75 mm, ou idéalement d'approximativement 72 mm.

7. Dispositif antironflement (1) selon l'une quelconque des revendications précédentes, dans lequel la longueur horizontale de l'insert (20) telle que mesurée à partir de la partie de base (21) vers l'extrémité opposée est dans la plage de 55 à 70 mm, de préférence dans la plage de 60 à 65 mm, ou idéalement d'approximativement 62 mm.

8. Dispositif antironflement (1) selon l'une quelconque des revendications précédentes, dans lequel la largeur horizontale de l'insert (20) est dans la plage de 55 à 75 mm, de préférence dans la plage de 60 à 70 mm, ou idéalement d'approximativement 75 mm.

9. Dispositif antironflement (1) selon la revendication 1, dans lequel le diamètre du trou central (26) dans la partie de base (21) est dans la plage de 25 à 40 mm, de préférence dans la plage de 30 à 35 mm, ou idéalement d'approximativement 32 mm.

10. Dispositif antironflement (1) selon la revendication 1, dans lequel la partie de base (21) comprend la forme d'une courbe convexe vers l'extérieur balayée selon un modèle elliptique ou ovale, la courbe dans une extrémité passant vers une troisième surface (24) de l'insert (20) et dans l'autre extrémité vers l'évidement (22) avec le trou (26) fournissant la surface de contact structurale en forme de tore ou toroïdale.

11. Dispositif antironflement (1) selon l'une quelconque des revendications précédentes, dans lequel le harnais (10) comprend deux sangles allongées (13a, 13b) fixées à chaque côté latéral de la partie de retenue (11), dans lequel lesdites sangles sont configurées pour maintenir le dispositif antironflement (1) en place autour du cou de l'utilisateur.

12. Dispositif antironflement (1) selon l'une quelconque des revendications précédentes, dans lequel le harnais (10) comprend deux sangles de retenue d'insert (12a, 12b), lesdites sangles (12a, 12b) étant configurées pour maintenir l'insert (20) en place dans la partie de retenue (11).
